# EUROPÄISCHE PATENTSCHRIFT

(11) **EP 3 386 564 B1**
(45) Veröffentlichungstag und Bekanntmachung des Hinweises auf die Patenterteilung: **10.06.2020**
(21) Anmeldenummer: 16801998.2
(22) Anmeldetag: 17.11.2016
(51) Int. Cl.: A61M 5/31, A61M 5/315, A61M 5/20

(54) **INJEKTIONSVORRICHTUNG ZUR INJEKTION DOSIERTER MENGEN EINES FLÜSSIGEN THERAPEUTIKUMS**
INJECTION DEVICE FOR INJECTING DOSED AMOUNTS OF A LIQUID THERAPEUTIC AGENT
DISPOSITIF D'INJECTION PERMETTANT D'INJECTER DES QUANTITÉS DOSÉES D'UN FLUIDE THÉRAPEUTIQUE

(30) Priorität: 09.12.2015 DE 102015121409
(43) Veröffentlichungstag der Anmeldung: 17.10.2018
(73) Patentinhaber: Emperra GmbH E-Health Technologies, 14469 Potsdam (DE)
(72) Erfinder: BENTRUP, Markus, 10629 Berlin (DE); SCHILDT, Janko, 14467 Potsdam (DE)
(74) Vertreter: Gulde & Partner
(86) Internationale Anmeldenummer: PCT/EP2016/078017
(87) Internationale Veröffentlichungsnummer: WO 2017/097560

(56) Entgegenhaltungen:
- EP-A1- 1 462 134
- EP-A2- 3 167 393
- WO-A1-2007/088444
- WO-A1-2010/098927
- WO-A1-2013/120778
- WO-A1-2014/173434
- WO-A1-2015/076745
- WO-A2-2016/007935
- US-A1- 2015 246 179

## Beschreibung

Die Erfindung betrifft eine Injektionsvorrichtung zur Injektion dosierter Mengen eines flüssigen Therapeutikums mit den im Oberbegriff des Anspruchs 1 genannten Merkmalen.

Viele Arzneimittel (Therapeutika) müssen in den Körper injiziert werden. Dies gilt vor allem für solche Arzneimittel, die bei oraler Gabe inaktiviert werden oder an Wirksamkeit entscheidend verlieren. Zu diesen Arzneimitteln zählen insbesondere Proteine, beispielsweise Insulin, Kohlehydrate, zum Beispiel Heparin, Antikörper oder die meisten Impfstoffe. Zur Injektion in den Körper werden überwiegend Spritzen, Medikamentenstifte (Pens) oder Medikamentenpumpen verwendet.

Bei der Insulintherapie, insbesondere bei der intensivierten, konventionellen Insulintherapie, und der konventionellen Insulintherapie, wird Insulin nicht in konstanten Mengen appliziert. Bei der konventionellen Insulintherapie wird zu bestimmten Tageszeiten Insulin appliziert. Der Tagesablauf des Patienten richtet sich nach diesen Zeiten. Bei der intensivierten, konventionellen Insulintherapie wird ein Grundbedarf an Insulin bereitgestellt, oft durch ein langsam und lang anhaltend wirkendes Insulin, das Basalinsulin.

Zu den Mahlzeiten wird schnell wirkendes Insulin gespritzt. Die Dosis des schnell wirkenden Insulins richtet sich im Wesentlichen nach den aufgenommenen Kohlehydraten. Die Dosis wird also in Abhängigkeit von den äußeren Umständen spezifisch ausgewählt. Solche Umstände sind beispielsweise die Tageszeit, der Bewegungsumfang, die Ernährung und dergleichen.

Diabetes mellitus kann schwerwiegende Langzeitfolgen und Körperschädigungen haben. Diese können durch eine angepasste Insulintherapie, vorzugsweise eine intensivierte, konventionelle Insulintherapie, wesentlich gemindert werden. Eine falsche Dosierung kann aber auch kurzzeitige Folgen wie Hypoglykämie-Zustände haben. Eine möglichst gute Anpassung der Dosis an die jeweiligen Umstände ist daher besonders erstrebenswert.

Aus diesem Grund werden die Diabetes-mellitus-Patienten zur genauen Protokollführung über ihre Lebensgewohnheiten und verabreichten Dosen an Insulin angehalten. Ein solches Protokoll umfasst üblicherweise den gemessenen Blutzuckerwert, die aufgenommene Menge an Kohlehydraten, die gespritzte Insulindosis sowie das Datum und die Uhrzeit. Aus dem Protokoll kann der behandelnde Arzt oder der Patient die jeweilige Dosis ermitteln oder anpassen. Es kommt also insbesondere auch auf eine hohe Dosiergenauigkeit an Insulin an.

Eine weit verbreitete Injektionsvorrichtung zur Injektion dosierter Mengen von Insulin ist der sogenannte Insulinpen. Im Unterschied zu Insulinspritzen wird beim Insulinpen ein wechselbarer Medikamentenbehälter eingesetzt. Dieser Behälter, auch Karpule bzw. Ampulle genannt, wird vom Hersteller mit dem Insulin befüllt ausgeliefert und vor Gebrauch in den Insulinpen eingesetzt. Bei Inbetriebnahme des Pens durchsticht eine Nadel die Dichtscheibe der Ampulle und realisiert bei der Applikation des Insulins die parenterale Injektion der vorgewählten Dosis. Ein Injektions- und Auslösemechanismus generiert während der Injektion einen Injektionshub, der den Vorschub eines Kolbens bzw. Stopfens in der Ampulle bewirkt und die Abgabe der vorgewählten Dosis in das Zielgewebe bedingt. Der Mechanismus besteht meist aus einer Kolbenstange mit einer dem Ampullenstopfenhub entsprechenden Baulänge.

Bekannte Insulinpens ähneln äußerlich einem dickeren Kugelschreiber. Sie umfassen ein Gehäuse, in dem die das Insulin enthaltende Ampulle aufgenommen werden kann. Die Ampulle ist gewöhnlich auswechselbar. Es sind aber auch Anordnungen bekannt, die als Einweg-Pens ausgebildet sind. Die Ampullen und deren Inhalt, Abmessungen und Handhabungen sind nicht normiert. Eine Ampulle eines Herstellers kann daher im Regelfall nicht in den Pen eines anderen Herstellers eingesetzt werden.

Aus EP 2 414 009 B1 ist ein Insulinpen bekannt, der eine Adapteranordnung zur Anpassung an Ampullen unterschiedlicher Abmessungen und Inhalte ermöglicht.

Ein Pen umfasst eine Dosiereinrichtung. An einem Dosierknopf wird die erforderliche Dosis eingestellt. Diese wird dann mittels einer Injektionseinrichtung, die nadelbehaftet oder nadellos ausgebildet sein kann, in das subkutane Fettgewebe gespritzt. Es sind Insulinpens bekannt, bei denen die eingestellte Dosis statt mit einer mechanischen Anzeige auf dem Dosierknopf auf einem Display angezeigt wird. Das Display wird mit einer im Insulinpen integrierten Spannungsquelle mit Energie versorgt. Der Patient kann die Dosis einstellen und in seinem Diabetikertagebuch notieren.

Es sind Insulinpens bekannt, bei denen die Protokollführung automatisch in einer in den Insulinpen integrierten Erfassungseinrichtung erfolgt. Diese ist über eine Datenverbindung, die drahtgebunden oder drahtlos ausgeführt sein kann, mit einer Datenverarbeitungseinheit verbindbar. Hinsichtlich Aufbau und Funktion eines derartigen Insulinpens wird auf die Offenbarung von WO 2013/079644 A1 verwiesen.

Insulinpens werden in einmalig verwendbare "disposable" und mehrfach verwendbare "reusable" Insulinpens eingeteilt. Bei einmalig verwendbaren Insulinpens bilden die Ampulle und die Dosiermechanik eine vom Hersteller vorgefertigte Einheit und werden nach Entleerung der Ampulle gemeinsam entsorgt. Eine Wiederverwendung der Dosiermechanik ist nicht vorgesehen. Mehrfach verwendbare Insulinpens stellen erhöhte Anforderungen an den Anwender. So muss beim Wechsel der Ampulle die Kolbenstange in die Startposition zurückgesetzt werden. Dies geschieht modellabhängig durch das Drehen bzw. Schieben der Kolbenstange bei gleichzeitiger Aktivierung einer Sonderfunktion in der Dosiermechanik.

Mehrfach verwendbare Insulinpens werden weiter unterteilt in manuelle und halbautomatische Insulinpens. Bei manuellen Insulinpens betätigt der Anwender mit Fingerkraft einen Injektionsknopf und bestimmt so Dauer und Verlauf der Injektion. Bei halbautomatischen Insulinpens hingegen wird vor Benutzung manuell eine Feder gespannt, die die notwendige Injektionsenergie speichert. Im eigentlichen Injektionsvorgang wird die Feder vom Anwender entriegelt.

Vor der eigentlichen Injektion ist eine Entlüftung der Injektionsvorrichtung erforderlich. Diese Entlüftung dient einerseits dazu, dass tatsächlich nur das Therapeutikum (Insulin) an den gewünschten Applikationsort gelangt. Andererseits dient die Entlüftung der Sicherstellung einer exakten Dosierung des zu injizierenden Therapeutikums. Der Anwender der Injektionsvorrichtung ist also angehalten, vor jeder Injektion eine manuelle Entlüftung der Injektionsvorrichtung durchzuführen. Dies erfolgt durch Auslösen der Injektionsvorrichtung vor der eigentlichen Injektion.

Hierbei ist nachteilig, dass die manuelle Entlüftung durch den Patienten fehlerbehaftet sein kann. Werden die Injektionsvorgänge in einer Erfassungseinrichtung automatisch aufgezeichnet, ist oft unklar, ob es sich um eine tatsächliche Injektion oder um eine Entlüftung der Injektionsvorrichtung handelt. Hierdurch sind die Aufzeichnungen fehlerbehaftet und die entsprechende Auswertung der tatsächlich injizierten Insulindosen kann fehlerbehaftet sein.

DE 696 25 498 T2 offenbart eine Anordnung von elektronisch gesteuerten Injektionsvorrichtungen bei denen ein Positionssensor vorgesehen ist. Der Positionssensor gibt eine der Orientierung der Vorrichtung entsprechende Signale ab. Hierdurch soll erreicht werden, dass eine Vorwärtsbewegung einer Kolbenstange nur möglich gemacht wird, wenn die Längsachse der Injektionsvorrichtung in einer bestimmten Richtung orientiert ist.

WO 2015/076745 A1, US 2015/0246179 A1 und EP 1 462 134 A1 offenbaren jeweils Injektionsvorrichtungen zur Injektion dosierter Mengen eines flüssigen Therapeutikums. Die Injektionsvorrichtungen weisen eine Sensoranordnung auf, die einen Näherungssensor umfasst, mittels dem ein Kontakt der Injektionsvorrichtung mit dem Körper einer die Injektion erhaltenden Person detektiert werden kann.

Die nachveröffentlichte EP 3 167 393 A2 offenbart eine Injektionsvorrichtung die sowohl einen Näherungssensor als auch einen Lagesensor umfasst.

Der Erfindung liegt die Aufgabe zugrunde, eine Injektionsvorrichtung der gattungsgemäßen Art zu schaffen, mit der in einfacher Weise mit größerer Genauigkeit erkannt werden kann, ob es sich bei dem Auslösen der Injektionsvorrichtung um eine Injektion oder um eine Entlüftung handelt.

Erfindungsgemäß wird diese Aufgabe durch eine Injektionsvorrichtung mit den in Anspruch 1 genannten Merkmalen gelöst. Dadurch, dass die Injektionsvorrichtung eine Sensoranordnung aufweist, die wenigstens einen Näherungssensor und einen Lagesensor umfasst, mittels denen detektiert wird wenn sowohl der Näherungssensor (44) als auch der Lagesensor (46) signalisieren, dass die Injektionsvorrichtung (10) waagerecht ist und eine Annäherung an die Haut einer zu injizierenden Person vorliegt, ob das Therapeutikum an den Applikationsort oder einen vom Applikationsort abweichenden Ort gelangt ist, wobei der Näherungssensor (44) ein kapazitiver Sensor und/oder ein optischer Sensor und/oder ein Ultraschallsensor ist, ist vorteilhaft möglich, automatisch zu erkennen, ob eine tatsächliche Injektion oder eine Entlüftung erfolgte. Diese Information wird der Erfassungseinrichtung der Injektionsvorrichtung zugeführt, so dass bei der Protokollführung berücksichtigt werden kann, dass eine Entlüftung vorliegt und keine Injektion. Dies erhöht die Qualität der auszuwertenden Protokolle, so dass eine genauere Dosierung des Therapeutikums erfasst bzw. eingestellt werden kann.

Da die Injektionsvorrichtung einen Näherungssensor und einen Lagesensor umfasst, wird vorteilhaft möglich, über den Lagesensor zu unterscheiden, ob sich die Injektionsvorrichtung in der senkrechten oderhorizontalen Position befindet und über den Näherungssensor zu unterscheiden, ob die

Injektionsvorrichtung auf der Haut der zu injizierenden Person aufsitzt oder von dieser noch entfernt ist. Wenn sowohl der Näherungssensor als auch der Lagesensor signalisieren, dass die Injektionsvorrichtung waagerecht ist und eine Annäherung an die Haut der zu injizierenden Person vorliegt, kann von einer erfolgreichen Injektion ausgegangen werden.

Unter Näherungssensor wird ein Sensor verstanden, der auf Annäherung, das heißt ohne direkten Kontakt, berührungsfrei reagiert. Durch Integration eines derartigen Näherungssensors in die Injektionsvorrichtung kann detektiert werden, ob die Injektionsvorrichtung bei Auslösung der Injektion in der Nähe eines Körpers, an dem der an sich gewünschte Applikationsort liegt, sich befindet oder die Injektion in die Luft erfolgt. Für den Fall, dass die Injektion in die Luft erfolgt, wird auf Entlüftung erkannt. In dem Fall, wo die Injektion, bei Auslösung des Näherungssensors, an dem Injektionsort erfolgt, wird auf eine gewünschte Injektion des Therapeutikums erkannt.

Bei dem Näherungssensor handelt es sich erfindungsgemäß um kapazitive Näherungssensoren, optische Näherungssensoren oder Ultraschall-Näherungssensoren. Diese sind alle geeignet, die Annäherung der Injektionsvorrichtung an einen menschlichen Körper zu detektieren.

Die Empfindlichkeit der Sensoren, das heißt der Lagesensoren und der Näherungssensoren, ist hierbei so eingestellt, dass auch noch bestimmte Winkelabweichungen von der Horizontalen bzw. bestimmte Mindestabstände bzw. Höchstabstände von der zu injizierenden Person erfasst werden. Insgesamt wird somit eine Injektionsvorrichtung bereitgestellt, mit der automatisch sicher erkannt werden kann, ob es sich um einen Entlüftungsvorgang oder einen Injektionsvorgang bei Auslösen der Injektionsvorrichtung handelt.

Weitere bevorzugte Ausgestaltungen der Erfindung ergeben sich aus den übrigen, in den Unteransprüchen genannten Merkmalen.

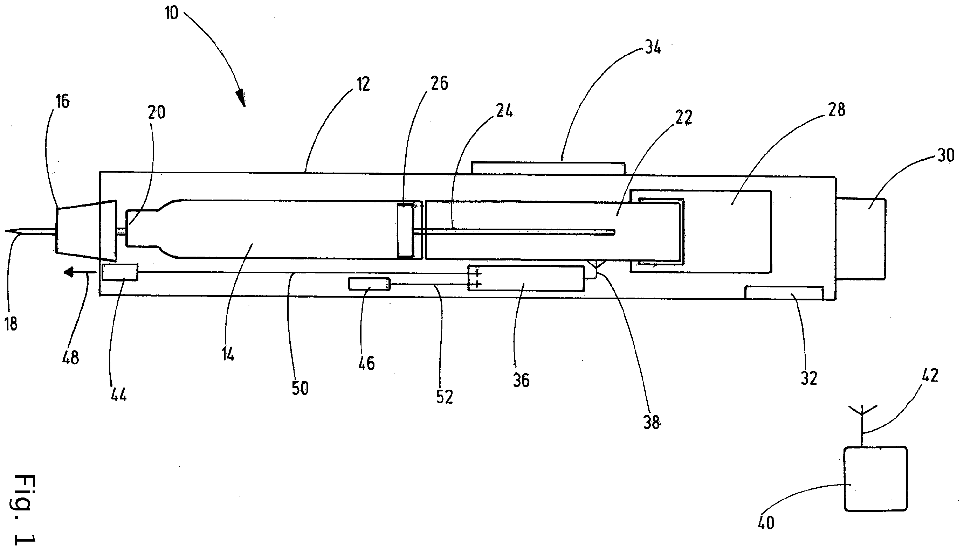

Die Erfindung wird nachfolgend in einem Ausführungsbeispiel anhand der dazugehörigen Zeichnung, die schematisch eine erfindungsgemäße Injektionsvorrichtung zeigt, näher erläutert. Die Figur zeigt schematisch eine insgesamt mit 10 bezeichnete Injektionsvorrichtung. Aufbau und Funktion von Injektionsvorrichtungen 10 sind allgemein bekannt, so dass im Rahmen der vorliegenden Beschreibung hierauf nicht näher eingegangen wird. Hierbei kann es sich beispielsweise um eine nadelbehaftete oder eine nadellose Injektionsvorrichtung handeln.

Auch kann es sich um eine einmalig oder mehrfach verwendbare Injektionsvorrichtung handeln. Darüber hinaus kann die Injektionsvorrichtung mit oder ohne Adapter zur Aufnahme unterschiedlicher Ampullen verschiedener Hersteller ausgestattet sein.

Die Injektionsvorrichtung 10 besitzt ein Gehäuse 12, innerhalb dem eine Aufnahmeeinrichtung 14 zur Aufnahme eines zu injizierenden Therapeutikums, nachfolgend wird von Insulin ausgegangen, angeordnet ist. Bei der Aufnahmeeinrichtung 14 kann es sich um eine Ampulle bzw. Karpule handeln.

Die Injektionsvorrichtung 10 umfasst ferner eine Applikationseinrichtung 16 zur Überführung des Insulins an einen Injektionsort (Applikationsort). Der Injektionsort ist beispielsweise eine Hautpartie

eines Patienten. Die Applikationseinrichtung 16 kann hierzu eine Pinnadel 18 besitzen, die in die Haut des Patienten eingestochen wird. Die Applikationseinrichtung 16 ist austauschbar an dem Gehäuse angeordnet und durchsticht mit dem Pin 18 eine Membran 20 der Aufnahmeeinrichtung 14.

Die Injektionsvorrichtung 10 umfasst ferner eine Dosiereinrichtung 22, die ein Betätigungselement 24 aufweist, das mit einem Stopfen 26 der Aufnahmeeinrichtung 14 in Wirkkontakt steht.

Der Dosiereinrichtung 22 ist eine Auslöseeinrichtung 28 zugeordnet. Die Auslöseeinrichtung 28 wirkt mit der Dosiereinrichtung 22 zusammen.

Die Injektionsvorrichtung 10 umfasst ferner einen Dosierknopf 30, über den die zu injizierende Dosis an Insulin eingestellt werden kann. Ferner ist ein Auslöseknopf 32 vorgesehen, der mit der Auslöseeinrichtung 28 wirkverbunden ist.

Die Injektionsvorrichtung 10 umfasst ferner ein Display 34, das mit einem Anzeigefeld ausgestattet ist.

Die Injektionsvorrichtung 10 umfasst ferner eine Erfassungseinrichtung 36, die über eine Schnittstelle 38 mit einer hier lediglich angedeuteten Datenverarbeitungsanlage 40 verbindbar ist. Die Datenverarbeitungsanlage 40 verfügt ebenfalls über eine Schnittstelle 42, die mit der Schnittstelle 38 kommunizieren kann. Die Verbindung kann hierbei drahtgebunden oder drahtlos erfolgen.

Die Injektionsvorrichtung 10 umfasst ferner einen Näherungssensor 44 und einen Lagesensor 46. Eine Detektionsrichtung des Näherungssensors 44 liegt in Längserstreckung der Injektionsvorrichtung 10, hier mit einem Pfeil 48 angedeutet.

Der Lagesensor 46 besitzt eine sensitive Einrichtung, beispielsweise eine entsprechend gelagerte seismische Masse, mit der erkannt wird, ob sich die Injektionsvorrichtung 10 in einer waagerechten Position, wie in der Figur gezeigt, oder in einer hierzu abweichenden senkrechten Position befindet. Die seismische Masse kann so ausgelegt sein, dass die waagerechte Position auch erkannt wird, wenn es eine Abweichung von beispielsweise in einem Winkelbereich von ±30° zu einer Horizontalen gibt.

Sowohl der Näherungssensor 44 als auch der Lagesensor 46 sind über Verbindungsleitungen 50 bzw. 52 mit der Erfassungseinrichtung 36 verbunden. Sowohl der Lagesensor 46 als auch der Näherungssensor 44 und die Verbindungsleitungen 50 und 52 können in das Gehäuse 12 der Injektionsvorrichtung 10 integriert sein, so dass diese eine definierte Position in Bezug auf das Gehäuse 12 besitzen.

Die in der Figur dargestellte Injektionsvorrichtung 10 zeigt folgende Funktion:
Bei einem bestimmungsgemäßen Einsatz der Injektionsvorrichtung 10 ist vor der eigentlichen Injektion eine Entlüftung durchzuführen. Diese Entlüftung dient zum sicheren Entfernen von sich im Pin 18 befindlicher Luft. Hierzu wird eine geringe Dosis an Insulin durch den Pin 18 in die Luft injiziert.

Für eine gewollte Injektion wird mittels des Dosierknopfes 30 die zu dosierende Menge an Insulin eingestellt. Die eingestellte Menge ist am Display 34 ablesbar und somit kontrollierbar. Nach Aufsetzen der Injektionsvorrichtung 10 mit dem Pin 18 der Applikationseinrichtung 16 auf die Haut des zu behandelnden Patienten wird der Auslöseknopf 32 betätigt. Hierauf wird mittels der Auslöseeinrichtung 28 der Injektionsvorgang ausgelöst, indem das Betätigungselement 24 der Dosiereinrichtung 22 mit einer Vortriebskraft beaufschlagt wird. Das Betätigungselement 24 verdrängt hierdurch den Stopfen 26 innerhalb der Aufnahmeeinrichtung 14, so dass die gewünschte eingestellte Dosis an Insulin über die Applikationseinrichtung 16 injiziert werden kann. Ein derartiger Aufbau und eine derartige Funktion der Injektionsvorrichtung 10 sind an sich bekannt.

Mittels des Näherungssensors 44 kann während einer Injektion erkannt werden, ob sich in der Injektionsrichtung 48 ein Gegenstand, hier die Haut der die Injektion erhaltenden Person, befindet. Ist dies nicht der Fall, wird die Injektion als Entlüftung gewertet und von der Erfassungseinrichtung 36 entsprechend registriert und gespeichert. Befindet sich die Haut im Detektionsfeld des Näherungssensors 44, wird die Injektion als erfolgreich erfasst und durch die Erfassungseinrichtung 36 entsprechend gespeichert.

Mittels des Lagesensors 46 kann zusätzlich erkannt werden, ob sich die Injektionsvorrichtung 10 in einer typischen Injektionsposition, nämlich in einer annähernd waagerechten bzw. horizontalen Position befindet.

Insgesamt wird mit der erfindungsgemäßen Injektionsvorrichtung 10 erreicht, dass die Dosiergenauigkeit erhöht wird. Etwaige Entlüftungen der Injektionsvorrichtung 10 werden sicher automatisch auch als solche erkannt und über die Erfassungseinrichtung 36 entsprechend protokolliert. Bei der späteren Auswertung der Protokolle durch einen behandelnden Arzt kann somit auf die tatsächlich applizierte Menge an Insulin genauer abgestellt werden.

### Bezugszeichen

- 10: Injektionsvorrichtung
- 12: Gehäuse
- 14: Aufnahmeeinrichtung
- 16: Applikationseinrichtung
- 18: Pin
- 20: Membran
- 22: Dosiereinrichtung
- 24: Betätigungselement
- 26: Stopfen
- 28: Auslöseeinrichtung
- 30: Dosierknopf
- 32: Auslöseknopf
- 34: Display
- 36: Erfassungseinrichtung
- 38: Schnittstelle
- 40: Datenverarbeitungsanlage
- 42: Schnittstelle
- 44: Näherungssensor
- 46: Lagesensor
- 48: Pfeil/Injektionsrichtung
- 50: Verbindungsleitung
- 52: Verbindungsleitung

## Patentansprüche

1. Injektionsvorrichtung (10) zur Injektion dosierter Mengen eines flüssigen Therapeutikums,
mit einer Aufnahmeeinrichtung (14) für das Therapeutikum, einer Applikationseinrichtung (16) zum Überführen des Therapeutikums an einen Applizierungsort, einer Dosiereinrichtung (22) zum Überführen des Therapeutikums von der Aufnahmeeinrichtung (14) zu der Applikationseinrichtung (16), einer Auslöseeinrichtung (28) zur Aktivierung der Dosiereinrichtung (22) sowie einer Erfassungseinrichtung (36) zum Erfassen der applizierten Menge des Therapeutikums, mit einer Sensoranordnung, die wenigstens einen Näherungssensor (44) und einen Lagesensor (46) umfasst, mittels denen detektiert wird, wenn sowohl der Näherungssensor (44) als auch der Lagesensor (46) signalisieren, dass die Injektionsvorrichtung (10) waagerecht ist und eine Annäherung an die Haut einer zu injizierenden Person vorliegt, ob das Therapeutikum an den Applizierungsort oder einen vom Applizierungsort abweichenden Ort gelangt ist, wobei der Näherungssensor (44) ein kapazitiver Sensor und/oder ein optischer Sensor und/oder ein Ultraschallsensor ist.

2. Injektionsvorrichtung (10) nach Anspruch 1,
**dadurch gekennzeichnet, dass** der Näherungssensor (44) und/oder der Lagesensor (46) in ein Gehäuse (12) der Injektionsvorrichtung (10) integriert sind.

3. Injektionsvorrichtung (10) nach einem der vorhergehenden Ansprüche,
**dadurch gekennzeichnet, dass** die Erfassungsvorrichtung (36) mit einer Datenverarbeitungsanlage (40) verbindbar ist.

## Claims

1. An injection device (10) for injecting dosed quantities of a liquid therapeutic agent, having a receiving member (14) for the therapeutic agent, an application member (16) for transferring the therapeutic agent to an application site, a dosing member (22) for transferring the therapeutic agent from the receiving member (14) to the application member (16), a release member (28) for activating the dosing member (22), and a detection member (36) for detecting the applied quantity of the therapeutic agent, having a sensor assembly comprising at least one proximity sensor (44) and one position sensor (46) by means of which, if both the proximity sensor (44) and the position sensor (46) signal that the injection device (10) is horizontal and that a person to be injected is being approached, it is detected whether or not the therapeutic agent has reached the application site or a site differing from the application site, wherein the proximity sensor (44) is a capacitive sensor and/or an optical sensor and/or an ultrasonic sensor.

2. The injection device (10) according to Claim 1,
**characterized in that** the proximity sensor (44) and/or the position sensor (46) are integrated into a case (12) of the injection device (10).

3. The injection device (10) according to any one of the preceding claims, **characterized in that** the detection device (36) is connectable to a data processing system (40).

## Revendications

1. Dispositif d'injection (10) pour injecter des quantités dosées d'un agent thérapeutique liquide, doté d'un réceptacle (14) de l'agent thérapeutique, d'un applicateur (16) pour transporter l'agent thérapeutique sur un site d'application, d'un doseur (22) pour acheminer l'agent thérapeutique du réceptacle (14) jusqu'à l'applicateur (16), d'un actionneur (28) pour activer le doseur (22) ainsi que d'un dispositif de détection (36) pour enregistrer la quantité appliquée de l'agent thérapeutique, d'un ensemble de capteurs comprenant au moins un capteur de proximité (44) et un capteur de position (46), au moyen desquels est détecté quand tant le capteur de proximité (44) que le capteur de position (46) signalent que le dispositif d'injection (10) est horizontal et qu'il s'approche de la peau d'une personne devant recevoir l'injection, si l'agent thérapeutique est parvenu au site d'application ou à un endroit s'écartant du site d'application, dans lequel le capteur de proximité (44) est un capteur capacitif et/ou un capteur optique et/ou un capteur ultrasonique.

2. Dispositif d'injection (10) selon la revendication 1,
**caractérisé en ce que** le capteur de proximité (44) et/ou le capteur de position (46) sont intégrés dans un boîtier (12) du dispositif d'injection (10).

3. Dispositif d'injection (10) selon l'une des revendications précédentes, **caractérisé en ce que** le dispositif de détection (36) peut être raccordé à une installation de traitement des données (40).
